# EUROPÄISCHE PATENTSCHRIFT

(11) **EP 2 419 007 B1**
(45) Veröffentlichungstag und Bekanntmachung des Hinweises auf die Patenterteilung: **20.04.2016**
(21) Anmeldenummer: 10717510.1
(22) Anmeldetag: 30.03.2010
(51) Int. Cl.: A61B 5/01, A61B 90/30

(54) **VERFAHREN UND EINRICHTUNG ZUR INTRAOPERATIVEN BILDGEBUNG VON GEHIRNAREALEN**
METHOD AND DEVICE FOR INTRAOPERATIVE IMAGING OF BRAIN AREAS
PROCÉDÉ ET DISPOSITIF D'IMAGERIE INTRAOPÉRATOIRE DE ZONES DU CERVEAU

(30) Priorität: 17.04.2009 DE 102009018633
(43) Veröffentlichungstag der Anmeldung: 22.02.2012
(73) Patentinhaber: Technische Universität Dresden, 01069 Dresden (DE)
(72) Erfinder: STEINER, Gerald, 08340 Schwarzenberg (DE); KOCH, Edmund, 01307 Dresden (DE); SCHACKERT, Gabriele, 01099 Dresden (DE); KIRSCH, Matthias, 01307 Dresden (DE)
(74) Vertreter: Hempel, Hartmut
(86) Internationale Anmeldenummer: PCT/DE2010/000398
(87) Internationale Veröffentlichungsnummer: WO 2010/118731

(56) Entgegenhaltungen:
- WO-A2-2007/061807
- GORBACH A M ET AL: "Intraoperative infrared imaging of brain tumors" JOURNAL OF NEUROSURGERY, AMERICAN ASSOCIATION OF NEUROLOGICAL SURGEONS, US, Bd. 101, Nr. 6, 1. Dezember 2004 (2004-12-01), Seiten 960-969, XP009135485 ISSN: 0022-3085
- ATSUHIRO NAKAGAWA ET AL: "Intraoperative infrared brain surface blood flow monitoring during superficial temporal artery-middle cerebral artery anastomosis in patients with childhood moyamoya disease" CHILD'S NERVOUS SYSTEM, SPRINGER, BERLIN, DE LNKD- DOI:10.1007/S00381-008-0682-9, Bd. 24, Nr. 11, 12. Juli 2008 (2008-07-12) , Seiten 1299-1305, XP019656508 ISSN: 1433-0350
- STEPHAN B. SOBOTTKA, KATHRIN D. GEIGER, REINER SALZER, GABRIELE SCHACKERT, CHRISTOPH KRAFFT: "Suitability of infrared spectroscopic imaging as an intraoperative tool in cerebral glioma surgery" ANAL BIOANAL CHEM, Bd. 393, 16. Oktober 2008 (2008-10-16), Seiten 187-195, XP002589663

## Beschreibung

Die Erfindung betrifft ein Verfahren und eine Einrichtung zur intraoperativen Bildgebung von Gehirnarealen.

Mittels des Verfahrens zur intraoperativen Bildgebung von Gehirnarealen in der Neurochirurgie werden mit einer Bildaufnahmeeinrichtung und eines Bildauswertungsverfahrens Änderungen in der Wärmestrahlung des freiliegenden Gehirns erfasst und bildhaft dargestellt. Das Ziel der Erfindung ist die Lokalisation funktionaler und/oder pathologischer Areale von Gehimgewebe. Damit soll die operative Präzision während eines neurochirurgischen Eingriffs maßgeblich verbessert werden. Dabei kann das Verfahren ohne Beeinträchtigung des Patienten bzw. ohne Schädigung des untersuchten Gehimgewebes eingesetzt werden und den operativen Ablauf nicht beeinträchtigen.

Eines der größten Probleme in der modernen Neurochirurgie stellt die fehlende Möglichkeit einer schnellen und sicheren Identifizierung funktioneller kortikaler Areale während des operativen Eingriffs dar.
Die Lösung des Problems ist nicht durch die konventionelle Lichtmikrokopie möglich, d. h. der Neurochirurg hat ohne zusätzliche Informationen keine Möglichkeit, funktionale Areale oder viele Arten von Pathologien an der Gehirnoberfläche zu identifizieren. Gleiches gilt für viele Pathologien, die sich nicht offensichtlich von normalem Gehimgewebe unterscheiden. Bisher wurden daher adjunkte Verfahren eingesetzt, z.B. maßgeblich die präoperative Bildgebung, die Lokalisation mittels stereotaxie-basierter Verfahren inklusive der Neuronavigation sowie aufwändiger intraoperativer funktioneller Untersuchungen. Zu den intraoperativen Verfahren gehören elektrophysiologische Detektion des primären Motorkortex sowie der primär sensorischen Areale (Gyrus postcentralis, primäre Sehrinde) bis zur Operation am wachen Patienten, um höhere Gehimfunktionen, z.B. Verständnis, Lesen, Rechnen, Sprechen und Bewegungsplanung zu lokalisieren.

Zu den experimentellen Verfahren, die in der klinischen Erprobung sind, gehört ein Verfahren zur optischen Bildgebung intrinsischer Signale des Kortex (engl. Optical Imaging). Der Bildkontrast beruht auf Änderungen der optischen Eigenschaften des Gehimgewebes bei neuronaler Aktivität. Die Ursachen dieser Änderungen sind eine oder mehrere der folgenden physiologischen Änderungen bei Gehimaktivität: Änderung der Sauerstoffsättigung des Hämoglobins, Änderung der Gewebestreuung, Änderung des Blutvolumens oder Änderung der Charakteristik des Chromophors Cytochrome-Oxidase. So kann durch das Optical Imaging z.B. nach sensorischer Reizung des Armes (somato-sensorisch evozierte Potentiale) ein korrespondierendes aktiviertes Areal kortikal zugeordnet werden, wenn die Trepanation (Gehimschädeleröffnung) über diesem Gebiet zugänglich ist.

Die ortsaufgelöste Messung von Wärmestrahlung wird üblicherweise als Thermographie bezeichnet. Dieses Verfahren wird bislang nicht für Aufgaben routinemäßig in der Neurochirurgie eingesetzt. Die Thermographie ist ein hoch entwickeltes, sensitives bildgebendes Verfahren, mit welchem kleine Temperatur differenzen sichtbar gemacht werden können.
Wie in der Druckschrift Shevelev et al.: Thermoimaging of the brain, Journal of Neuroscience Methods, 46, 1993, S. 49-57 beschrieben, ist bereits vor mehreren Jahrzehnten versucht worden, an exponierten Gehirnarealen Pathologien, insbesondere ischämische Läsionen ("Gehirninfarkt", ischämischer Insult, engl. ischemic stroke) thermographisch zu identifizieren, da ein minderdurchblutetes Areal außerhalb der geschlossenen Kalotte eine lokal niedrigere Wärmeleistung aufweisen sollte. Die darin untersuchte Wärmeleistung des aktivierten durchbluteten Kortex kann durch einen erhöhten Metabolismus und einer konsekutiv erhöhten lokalen Durchblutung 0,001 Watt erreichen.

Die Thermographie basiert auf dem nach außen abgestrahlten infraroten Licht im Bereich von ca. 1200nm bis 2500nm Wellenlänge. Je nach Kameratyp, Filter und wärmesensitivem Bilddetektor kann dieser Bereich variieren. Die Thermographie ist nicht mit der intraprenchymatösen Temperaturmessung zu verwechseln, die auf der Implantation einer Sonde beruht, die an einer Position verankert wird und nur dort die Temperatur im Inneren des Gehirns misst.

Andere Verfahren zur Messung der intrakraniellen Temperaturverteilung stellen die Magnetresonanzspektroskopie, die Mikrowellenradiometrie und die Ultraschallthermometrie dar. Diese Verfahren können jedoch nicht oder nur mit großen Einschränkungen intraoperativ eingesetzt werden.

Bezüglich einer Lokalisierung von kortikalen funktionellen Arealen durch Thermographie wurde neben den genannten Verfahren zur Lokalisierung bestimmter funktioneller Areale, namentlich den primären Motorkortex und den primären sensorischen Kortex (Gyrus praecentralis und Gyros postcentralis) bereits vor zwanzig Jahren die Thermographie in analoger Weise zur Detektion normaler funktioneller Gehimareale eingesetzt.

Insbesondere in der Druckschrift Gorbach et al.: Intraoperative Infrared Functional Imaging of Human Brain, Annals of Neurology, 2003, 54, S. 297-309 ist die systematische Etablierung des Verfahrens zur Lokalisierung des primär motorischen und sensiblen Kortex beschrieben. Darin erfolgte eine Untersuchung von einundzwanzig Patienten, die intraoperativ einer Nervus medianus - Stimulation oder während einer Wachoperation verschiedenen Aufgaben unterzogen wurden, in welchen kortikalen Arealen eine gesteigerte Wärmeleistung zu verzeichnen war. Die Ergebnisse der Thermographie stimmen mit den Messungen durch konventionelle kortikale Reizung und elektrophysiologische Kartierung überein und dehnten sich zum Teil über diese Bereiche aus.

Eine Detektion von Gefäßmissbildungen ist in der Druckschrift Nakagawa et al.: Use of Intraoperative Dynamic Infrared Imaging with Detection Wavelength of 7-14µm in the Surgical Obliteration of Spinal Arteriovenous Fistula: Case Report and Technical Considerations, Minim Invas Neurosurg, 2004, 47, S. 136-139, Georg Thieme Verlag KG Stuttgart, New York beschrieben, wobei die Detektion von Gefäßmissbildungen im Rückenmark eines Patienten durch intraoperative Infrarotbildgebung erfolgte, die eine thermische Auflösung von 0,15 Kelvin aufwies. Bei der Detektion lag der Schwerpunkt nicht auf den thermischen Signalen, sondern auf der selektiven Blutflussmessung aufgrund des Infrarot-Signals des Blutes bei einer Wellenlänge von 7-14 µm. Nach Extirpation der Gefäßmissbildung konnte kein Flusssignal detektiert werden. Über eine thermographische Messung wurde nicht berichtet, weil eine Thermographie in diesem Fall unmöglich war, da die tiefe, von stark durchbluteten Wänden umgebene Resektionshöhle einen lokalen Wärmestau induzierte und ein hohes Rauschen verursachte.

Eine Anwendung des Nahinfrarot-Imaging zur Charakterisierung von Gewebe wird in der Druckschrift WO 2006/014736 A2 beschrieben, wobei insbesondere auf das Monitoring von Therapien eingegangenen wird. Es werden keine Vorgänge beschrieben, wie aus den thermografischen Bildern durch Nutzung multivariater Bildanalyseverfahren Informationen gewonnen werden können.

Ein thermisches Abbildungsverfahren wird in der Druckschrift GB 2311368 A beschrieben, wobei malignes Gewebe, insbesondere der Haut, anhand der veränderten Temperatur erkannt werden kann. Messungen von Gehimarealen zur Erkennung und Abgrenzung funktionaler Areale werden nicht beschrieben.

Ein optisches Abbildungssystem für die intraoperativen Messungen am Gehirn wird in der Druckschrift US 005215095 A dargestellt. Das Abbildungssystem enthält explizit eine Signalverarbeitung, wobei aber nur optische Bilder im sichtbaren Spektralbereich registriert und verarbeitet werden.

Ein Abbildungssystem zur Erfassung kognitativer Funktionen wird in der Druckschrift US 2004/0082862 A1 beschrieben. Das zugehörige Verfahren wird nicht intraoperativ eingesetzt und dient ebenso wenig der Erkennung und Abgrenzung funktionaler oder pathologischer Areale.

In der Druckschrift WO 02/29700 A2 wird ein Verfahren zur intraoperativen gestützten Neurochirurgie beschrieben. Das bildgebende Verfahren beruht auf der Erhöhung des optischen Kontrastes.

Der Erfindung liegt die Aufgabe zugrunde, ein Verfahren und eine Einrichtung zur intraoperativen Bildgebung von Gehirnarealen anzugeben, die derart geeignet ausgebildet sind, dass funktionelle kortikale und/oder pathologische Areale dargestellt und abgegrenzt werden können. Dabei soll eine bildhafte Erfassung während eines operativen Eingriffs möglich sein und keine Schädigung oder Rückwirkung auf das Gewebe vorhanden sein. Zudem sollen die untersuchten Flächen der Gehimareale einer mathematischen Auswertung unterzogen werden und zugleich zeitliche Veränderungen der Gehirnareale anzeigen. Ziel der eingesetzten multivariaten Bildauswerteverfahren ist eine hochsensitive Erfassung minimaler Temperaturänderungen. Es sollen auch der Bildkontrast verbessert und wichtige Gebiete der Gehimoberfläche herausgehoben werden.

Die Aufgabe wird mit den Merkmalen der Patentansprüche 1 und 9 gelöst.
Das Verfahren zur intraoperativen Bildgebung von Gehirnarealen weist folgende Schritte auf:
- die Oberfläche eines Gehirnareals wird mittels einer Wärmebildkamera bildhaft erfasst,
- die Wärmebilder werden einige Sekunden bis wenige Minuten kontinuierlich als Bildsequenz aufgenommen,
- die Bildsequenz wird einer Einheit zur multivariaten Bildauswertung zugeführt und wird dort als Datenkubus einer multivariaten Auswertung unterzogen,
- die temperaturunterschiedlichen Bereiche der Oberfläche des Gehirnareals werden hervorgehoben,
- die Wärmebilder werden wahlweise in das jeweils zutreffende mikroskopische Bild eingespiegelt und/oder mit den präoperativen Bilddaten gemeinsam registriert.

Dazu wird eine Wärmebildkamera eingesetzt, die den Spektralbereich von 800nm bis zumindest 20000nm erfasst und zugleich unempfindlich für sichtbares Licht ist, wobei für höhere Temperaturen des zu untersuchenden Gehirnareals der Spektralbereich wahlweise über 20000nm hinausreichen kann.

Die Wärmebildkamera kann oberhalb des abzubildenden Gehirnareals positioniert werden.

Die Wärmebildkamera kann dabei so angebracht werden, dass sie unter Aufrechterhaltung der Sterilität in allen Raumrichtungen frei positioniert wird.

Die Wärmebildkamera wird separat an Haltevorrichtungen oder an weiteren intraoperativen Instrumenten, z.B. Navigationseinheit oder am Operationsmikroskop fixiert.

Die Wärmebildkamera kann aber auch in das Operationsmikroskop integriert werden.

Bei der Erfassung der Wärmestrahlung wird ausschließlich die gehirneigene Wärmestrahlung der Wärmebildkamera zugeführt.

Es können kontinuierlich Bilder von dem abzubildenden Gehirnareal mit einer Abtastfrequenz zwischen 0.5HZ und 50Hz aufgenommen und gespeichert werden.

Die Wärmebilder können sofort nach Abschluss der Aufnahmesequenz in einer Einheit zur multivariaten Bildauswertung ausgewertet werden, von der aus das berechnete Bild ausgegeben wird.

Während der multivariaten Bildauswertung werden Störungen der Aufnahmen erkannt und die gestörten Wärmebilder aus dem Datenkubus eliminiert.

Die Änderungen in der Basislinie können automatisch korrigiert durch eine lineare oder eine nichtlineare Basislinienkorrektur kompensiert werden.
Als Basislinie wird der Temperatur-Untergrund, d.h. ein konstanter Untergrund bei der kleinsten gemessenen Temperatur (z.B. 30°C) festgelegt, der sich durch eine langsame Veränderung der Temperatur infolge von äußeren Einflüssen (z.B. Erwärmung oder Abkühlung) oder durch die Wärmebildkamera selbst infolge von Drift ändern kann. Innerhalb der relativ kurzen Messzeiten von wenigen Minuten sind die Änderungen in sehr guter Näherung linear und können durch eine Zwei-Punkt-Anpassung der Basislinie korrigiert werden.

Die erfassten Wärmebilder können einer Hauptkomponentenanalyse, einem Verfahren der Clusteranalyse oder einem statistischen Klassifikationsverfahren zugeführt werden.

Des Weiteren können aktive Gehirnareale in einem Falschfarbenbild deutlich hervortreten.

Das Wärmebild kann einzeln oder fusioniert mit dem mikroskopischen Bild oder mit präoperativen Bilddaten dargestellt werden.

Die Bildsequenz der Wärmebilder kann in zeitlicher Abfolge sichtbar gemacht werden.

Zusammenfassend können folgende Verfahrensschritte vorgesehen werden:
- die aufgenommenen Wärmebilder werden in einer Einheit zur multivariaten Bildauswertung zugeführt und einem multivariaten Auswerteverfahren unterworfen, wobei ein Datenkubus von Wärmebildern erstellt wird,
- Auswertung der Wärmebilder nach Abschluss der Wärmebildsequenz,
- Berechnung der auszugebenden Bilder, wobei Störungen in der Aufnahme erkannt werden und die gestörten Wärmebilder aus dem Datenkubus entfernt werden,
- Korrektur der Änderungen in der Basislinie,
- Kompensation der Änderungen durch eine lineare oder eine nichtlineare Basislinienkorrektur, wobei das Wärmebild einzeln oder fusioniert mit dem mikroskopischen Bild oder mit präoperativen Bilddaten dargestellt wird.

Dabei können am abzubildenden Gehirnareal aufgezeichnete elektrophysiologische Messungen in die Wärmebilder zeitgenau eingespiegelt werden.

Der zeitliche Ablauf der elektrophysiologischen Stimulationen können zeitgenau mit den Wärmebildern dargestellt und beliebig oft wiederholbar gezeigt werden kann.

Das Verfahren kann zur Durchführung der Wärmebildanalyse nach Erstellung des Datenkubus von Wärmebildern folgende Schritte aufweisen:
- Herausfilterung von offensichtlichen Störungen aus den Wärmebildern des Datenkubus in einem ersten Schritt,
- pixelweise Datenvorbehandlung von zeitlichen Sequenzen in einem zweiten Schritt,
- Auswahl und Durchführung der multivariaten Auswertung und Klassifizierung der Bilddaten in einem dritten Schritt,
- Bildrekonstruktion in einem vierten Schritt,
- Kontrastverstärkung in einem fünften Schritt und
- Ausgabe/Anzeige des ausgewerteten Bildes in einem sechsten Schritt.

Zur Wärmebildanalyse kann im dritten Schritt der Auswertung und Klassifizierung eine überwachte Klassifizierung der Bilddaten mittels Trainingsdaten und/oder eine nichtüberwachte Klassifizierung durchgeführt werden.

Die Einrichtung zur intraoperativen Bildgebung von Gehirnarealen, deren abgestrahlte Wärmestrahlung mittels einer Detektion unter Einsatz des vorgenannten Verfahrens erfassbar ist,
enthält
folgende Baugruppen zur Erfassung der Wärmestrahlung
- eine Wärmebildkamera zur Aufnahme der vom Gehirnareal abgestrahlten Wärmestrahlung in Form eines Wärmestrahlenbündels,
- eine Bildverarbeitungseinheit, die der Wärmebildkamera nachgeordnet ist und in der die aufgenommenen Wärmebilder in einem Datenkubus stapelartig gespeichert sind,
   wobei in der Bildverarbeitungseinheit eine Einheit zur multivariaten Bildauswertung vorhanden ist, die
   o eine Einheit zur Entfernung von Störungen und Bildfehlern aus dem Datenkubus und
   o eine Einheit zur multivariaten Analyse des Datenkubus enthält,
      wobei von der Bildverarbeitungseinheit berechnete Bilder und Informationen aus dem Datenkubus der Wärmebilder zur Beobachtung erstellt werden, und
- Ausgabevorrichtungen für das Beobachten der Wärmebilder.

In der Einrichtung können zumindest ein Operationsmikroskop, das ein Linsensystem zur Abbildung eines vorgesehenen Gehirnareals mittels eines sichtbaren Lichtstrahlenbündels, das die vom Gehirnareal abgestrahlte Wärmestrahlung als Wärmestrahlenbündel enthält, und folgende weitere Baugruppen vorhanden sein:
- ein Spiegelsystem mit zumindest einem dichroitischen Teilerspiegel zur Transmission des sichtbaren Lichtstrahlenbündels und zur Ablenkung des im Strahlengang des sichtbaren Lichtstrahlenbündels befindlichen Wärmestrahlenbündels, wobei mittels des dichroitischen Teilerspiegels das Wärmestrahlenbündel aus dem sichtbaren Lichtstrahlenbündel abgelenkt wird,
- eine Wärmebildkamera zur Aufnahme des aus dem sichtbaren Lichtstrahlenbündel abgelenkten Wärmestrahlenbündels,
- eine Bildverarbeitungseinheit, die der Wärmebildkamera nachgeordnet ist und in der die aufgenommenen Wärmebilder in einem Datenkubus stapelartig gespeichert sind,
   wobei in der Bildverarbeitungseinheit eine Einheit zur multivariaten Bildauswertung vorhanden ist, die
   o eine Einheit zur Entfernung von Störungen und Bildfehlern aus dem Datenkubus und
   o eine Einheit zur multivariaten Analyse des Datenkubus enthält,
      wobei von der Bildverarbeitungseinheit berechnete Bilder und Informationen aus dem Datenkubus der Wärmebilder zur Beobachtung erstellt werden, und
- Ausgabevorrichtungen für das Beobachten der Bilder des Operationsmikroskops und für das Beobachten der Wärmebilder.

Das Spiegelsystem mit dem die Wärmebilder ablenkenden dichroitischen Teilerspiegel kann mindestens einen weiteren Spiegel zur nachfolgenden Umlenkung des Wärmestrahlenbündels zur Detektorfläche der Wärmebildkamera aufweisen.

Ein EEG-Gerät für elektrophysiologische Messungen kann in die Einrichtung eingebunden sein, wobei Elektroden des EEG-Gerätes im Bereich des abzubildenden Gehirnareals angebracht sind, mit denen der zugehörige EEG-Verlauf aufgezeichnet wird.

Die Ausgabevorrichtungen können zeitliche Darstellungen der Wärmebilder anzeigen.

Der Datenkubus von Wärmebildern weist eine Folge von zeitlich aufeinander folgend registrierten Wärmebildern auf.

In der Einheit zur Entfernung von Störungen und Bildfehlern aus dem Datenkubus können folgende Funktionen und Baugruppen realisiert sein:
- eine Clusteranalyse zur Trennung von gestörten und nicht gestörten Bildern,
- ein Filteralgorithmus, der Bilder mit einer physiologisch nicht möglichen schnellen Änderung in der Wärmestrahlung als Artefakt klassifiziert und eliminiert,
- ein Filter, der sämtliche Pixel eines Bildes unterhalb einer einzustellenden oder automatisch gewählten Temperaturgrenze aus dem Wärmebilddatensatz eliminiert,
- ein Rekonstruktionsalgorithmus, der vor allem flächenmäßig kleine Bildfehler durch mathematische Operationen, wie zum Beispiel Faltung, ausgleicht.

In der Einheit zur multivariaten Analyse des Datenkubus können folgende Funktionen realisiert sein:
- eine Hauptkomponentenanalyse,
- eine Koeffizientenanalyse,
- eine hierarchischen oder nichthierachischen Clusteranalyse,
- eine linearen Diskriminanzanalyse und ein Algorithmus zur Selektion relevanter Sequenzen,
- eine überwachten Klassifizierung unter Zuhilfenahme von Trainingsdaten, die in dem Algorithmus zur Selektion relevanter Sequenzen bereits gespeichert sind.

Ferner können auch Verfahren der künstlichen neuronalen Netze und genetische Algorithmen zur Anpassung der Klassifikation eingesetzt werden.

Die Wärmebildkamera kann separat an Haltevorrichtungen oder an weiteren intraoperativen Instrumenten, an einer Navigationseinheit oder am Operationsmikroskop fixiert sein.

Die Wärmebildkamera kann außerdem in das Operationsmikroskop integriert sein.

Der Abstand zwischen der Wärmebildkamera und dem abzubildenden Gehirnareal kann einen Dimensionierungsbereich zwischen 0,3m und 1m umfassen.

Die eingesetzte Wärmebildkamera kann ungekühlt sein und zumindest eine Temperatur-Empfindlichkeit von 0,07 Kelvin aufweisen.

Ein Vorteil der Erfindung besteht darin, dass die Oberfläche des frei zugänglichen Gehimgewebes berührungslos und rückwirkungsfrei durch die wärmestrahlungssensitive Kamera - Wärmebildkamera - erfasst wird. Die Position der Wärmebildkamera und deren Focuspunkt können in der Navigation oder ins Operationsmikroskop eingebunden werden, wobei eine Koregistrierung ermöglicht werden kann. Alternativ können die Wärmebilder unabhängig von Operationsmikroskop/Navigation aufgenommen werden, online oder nach post-processing in das Video-Bild integriert bzw. koregistriert werden oder mit den Daten eines Navigationssystems koregistriert werden. Die Hardware- und Software-Koppelung der Thermographie kann somit für die Operationsmikroskopanbindung, alternativ für die Navigationseinheit oder weitere intraoperative Bildgebungsmodalitäten (z.B: offenes MRT, CT, Ultraschall oder andere) genutzt werden.

Während einer bestimmten Zeit werden kontinuierlich thermographische Bilder - Wärmebilder - aufgezeichnet und einem speziellen Auswerteverfahren unterzogen.
Während der Messung ist die Wärmebildkamera ruhig in einer festen Position fixiert. Geringfügige Schwankungen durch übliche Erschütterungen oder Bewegungen des Gehirns, zum Beispiel durch den Puls, werden von der nachfolgenden Auswertung erkannt und entsprechend korrigiert. Die gewonnen Bilddaten liegen in der Form eines Datenkubus vor, der einem Filteralgorithmus unterzogen wird, der dazu dient, Störungen und Fremdeinflüsse heraus zu filtern. Spülungen und andere operative Eingriffe zeichnen sich immer durch eine sprunghafte Änderung der Wärmestrahlung von mindestens 1 K aus. Die Änderung der Wärmestrahlung wird pixelweise von dem Filteralgorithmus erkannt. Tritt diese Störung in mindestens 10% der Bildpunkte auf, so wird das Wärmebild aus dem Datenkubus entfernt. Andere kleinere Störungen werden durch vorgegebene Filteralgorithmen der Bildrestauration behoben. Nachfolgend wird der gefilterte Datenkubus einer Vorbehandlung unterzogen. Ziel der Datenvorbehandlung ist die Kompensation von zeitlich sehr langsamen Prozessen und Veränderungen, die nicht mit der Gehimaktivität in Verbindung stehen. Je nach Eigenschaften des Bilddatenkubus erfolgt eine Korrektur des Signaluntergrundes, eine pixelweise Normierung der zeitlichen Änderungen und/oder eine Zentrierung der Daten. Dadurch erfolgt eine Heraushebung schneller Änderungen der Wärmestrahlung. Die Quantifizierung und Klassifizierung von Bildarealen mit gleichen oder ähnlichen zeitlichen Änderungen der Wärmestrahlung erfolgt durch Verfahren der multivariaten Auswertung. Hier kommt zum Beispiel die Hauptkomponentenanalyse, Verfahren der Clusteranalyse oder Supported Vectormachines zum Einsatz. Weiterhin können überwachte Verfahren mit einer Klassifikation, beruhend auf einem Trainingsdatenkubus, eingesetzt werden.
Vorteilhafte Weiterbildungen und weitere Ausbildungen der Erfindung sind in weiteren Unteransprüchen angegeben.

Die Erfindung wird mittels eines Ausführungsbeispiels anhand mehrerer Zeichnungen näher erläutert.

Es zeigen:
- Fig. 1: eine schematische Darstellung einer erfindungsgemäßen Einrichtung zur intraoperativen Bildgebung von Gehimarealen,
- Fig. 2: eine Verarbeitungsschrittfolge nach der Aufnahme von Wärmebildern mittels der Wärmebildkamera innerhalb der Bildverarbeitungseinheit,

- Fig. 3: eine Darstellung der operativen Anordnung und des wärmebildkamerabezogenen Aufnahmebereiches und der zugehörigen Signalverarbeitungs- und Anzeigeverfahrensschritte innerhalb der erfindungsgemäßen Einrichtung,
- Fig. 4: Temperaturänderung-Zeit-Kurven als Aufnahmebilder von Oberflächenregionen des Gehirnareals, wobei
Fig. 4a eine Oberflächenregion mit erhöhter Temperatur und zugehörigem Temperaturverlauf und
Fig. 4b eine Oberflächenregion mit normaler Temperatur und zugehörigem Temperaturverlauf zeigen, und
- Fig. 5: einen Ablauf der Wärmebilddatenanalyse.

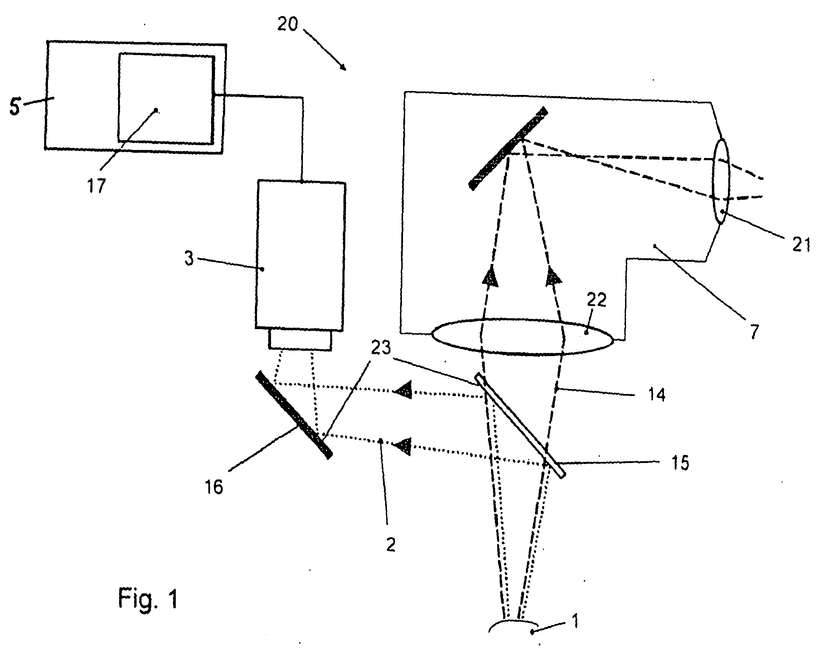

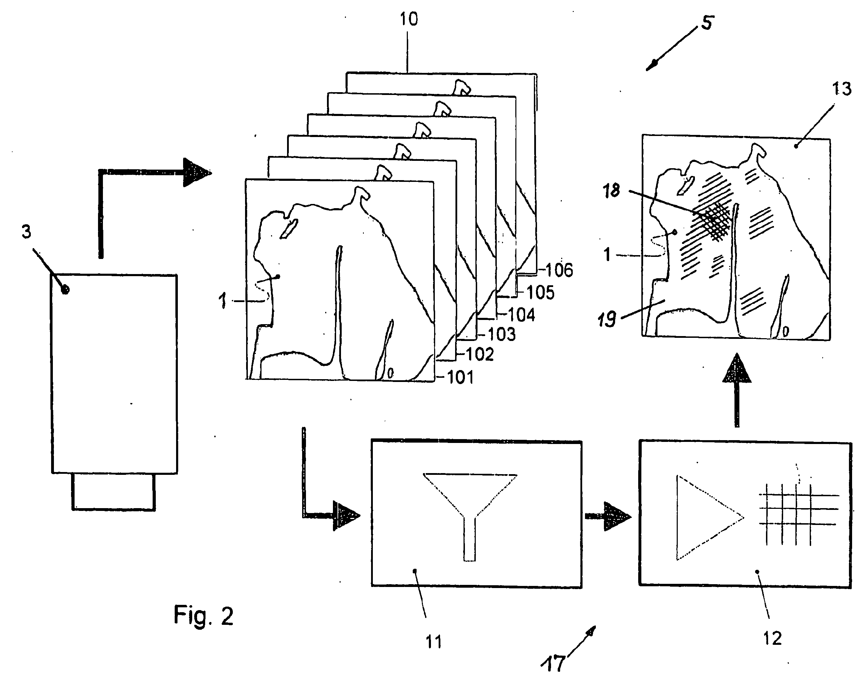

Im Folgenden werden Fig. 1 und 2 gemeinsam betrachtet.
Die Fig. 1 zeigt eine Ausführungsform der erfindungsgemäßen Einrichtung 20. Die Einrichtung 20 zur intraoperativen Bildgebung von Gehimarealen 1 enthält zumindest ein Operationsmikroskop 7, das ein Linsensystem 21, 22 zur Abbildung eines vorgesehenen Gehimareals 1 mittels eines sichtbaren Lichtstrahlenbündels 14, das die vom Gehimareal 1 abgestrahlte Wärmestrahlung als Wärmestrahlenbündel 2 enthält, aufweist.

Erfindungsgemäß sind in Verbindung mit dem Operationsmikroskop 7 folgende weitere Baugruppen vorhanden
- ein Spiegelsystem 23 mit zumindest einem dichroitischen Teilerspiegel 15 zur Transmission des sichtbaren Lichtstrahlenbündels 14 und zur Ablenkung des im Strahlengang des sichtbaren Lichtstrahlenbündels 14 befindlichen Wärmestrahlenbündels 2, wobei mittels des dichroitischen Teilerspiegels 15 das Wärmestrahlenbündel 2 aus dem sichtbaren Lichtstrahlenbündel 14 abgelenkt wird,
- eine Wärmebildkamera 3 zur Aufnahme des aus dem Lichtstrahlenbündel 14 abgelenkten Wärmestrahlenbündels 2,
- eine Bildverarbeitungseinheit 5, die der Wärmebildkamera 3 nachgeordnet ist und in der die aufgenommenen Wärmebilder 101, 102, 103, 104, 105, 106 in einem Datenkubus 10 (in Fig. 2) stapelartig gespeichert sind, wobei in der Bildverarbeitungseinheit 5 eine Einheit 17 zur multivariaten Bildauswertung vorhanden ist, die
   o eine Einheit 11 zur Entfernung von Störungen und Bildfehlern aus dem Datenkubus 10 und
   o eine Einheit 12 zur multivariaten Analyse des Datenkubus 10 enthält,
      wobei von der Bildverarbeitungseinheit 5 berechnete Bilder 13 und Informationen aus dem Datenkubus 10 der Wärmebilder 101, 102, 103, 104, 105, 106 zur Beobachtung erstellt werden, und
- Ausgabevorrichtungen 6, 6' für das Beobachten der Bilder des Operationsmikroskops 7 und für das Beobachten der Wärmebilder 101, 102, 103, 104, 105, 106.

In dieser Ausführungsform der Einrichtung 20 wird das sichtbare Licht 14 zusammen mit der Wärmestrahlung 2 aufgenommen. Der dichroitische Teilerspiegel 15 trennt die spektralen Bereiche, wobei die Wärmestrahlung 2 der wärmesensitiven Kamera 3 und das sichtbare Licht 14 dem Operationsmikroskop 7 getrennt zugeführt werden.
Ein Vorteil dieser Ausführungsform ist die Aufnahme identischer Gehimareale 1 mittels sichtbaren Lichts 14 und Wärmestrahlung 2.

Das Spiegelsystem 23 mit dem die Wärmebilder 101, 102, 103, 104, 105, 106 ablenkenden dichroitischen Teilerspiegel 15 weist einen weiteren Spiegel 16 zur nachfolgenden Umlenkung des Wärmestrahlenbündels 2 zur Aufnahmefläche der Wärmebildkamera 3 auf.

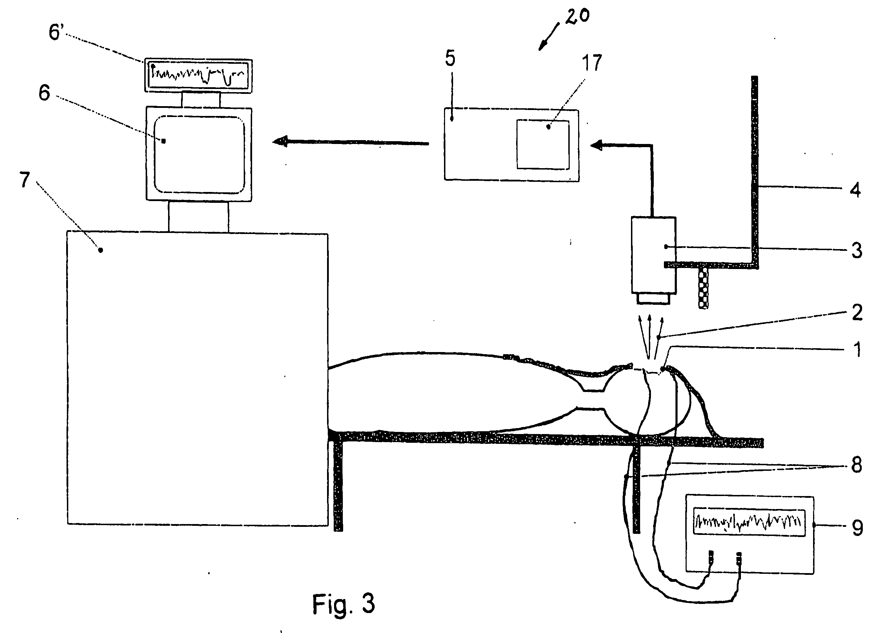

Ein in Fig. 3 dargestelltes EEG(Elektroenzephalogramm)-Gerät 9 für elektrophysiologische Messungen ist in die Einrichtung 20 eingebunden, wobei Elektroden 8 des EEG-Gerätes 9 im Bereich des abzubildenden Gehirnareals 1 angebracht sind, mit denen der zugehörige EEG-Verlauf aufgezeichnet wird.

Die Ausgabevorrichtungen 6, 6' zeigen zeitliche Darstellungen der Wärmebilder 101, 102, 103, 104, 105, 106 an.

Der Datenkubus 10 von Wärmebildern in Fig. 2 weist eine Folge von zeitlich aufeinander folgend registrierten Wärmebildern 101, 102, 103, 104, 105, 106 auf.

In der Einheit 11 zur Entfernung von Störungen und Bildfehlern aus dem Datenkubus 10 können folgende Funktionen und Baugruppen realisiert sein:
- eine Clusteranalyse zur Trennung von gestörten und nichtgestörten Bildern,
- ein Filteralgorithmus, der Bilder mit einer physiologisch nicht möglichen schnellen Änderung in der Wärmestrahlung als Artefakt klassifiziert und eliminiert,
- ein Filter, der sämtliche Pixel eines Bildes unterhalb einer einzustellenden oder automatisch gewählten Temperaturgrenze aus dem Wärmebilddatensatz eliminiert,
- ein Rekonstruktionsalgorithmus, der vor allem flächenmäßig kleine Bildfehler durch mathematische Operationen, wie zum Beispiel Faltung, ausgleicht.

In der Einheit 12 zur multivariaten Analyse des Datenkubus 10 können folgende Funktionen realisiert sein:
- eine Hauptkomponentenanalyse,
- eine Koeffizientenanalyse,
- eine hierarchischen oder nichthierachischen Clusteranalyse,
- lineare Diskriminanzanalyse und ein Algorithmus zur Selektion relevanter Sequenzen,
- einer gestützten Klassifizierung unter Zuhilfenahme von Trainingsdaten die in dem Algorithmus zur Selektion relevanter Sequenzen bereits gespeichert sind.

Ferner können auch Verfahren der künstlichen neuronalen Netze und genetische Algorithmen zur Anpassung der Klassifikation eingesetzt werden.

Es kann eine Wärmebildkamera 3 eingesetzt sein, die den Spektralbereich von 800 nm bis mindestens 20000 nm erfasst und zugleich unempfindlich für sichtbares Licht ist. Für höhere Temperaturen des zu untersuchenden Gehirnareals 1 kann der Spektralbereich über 20000nm hinausreichen.

Die Wärmebildkamera 3 kann separat an Haltevorrichtungen 4 oder an weiteren intraoperativen Instrumenten, an einer Navigationseinheit oder am Operationsmikroskop fixiert sein.

Die Wärmebildkamera 3 kann auch in das Operationsmikroskop 7 integriert sein.

Der Abstand zwischen der Wärmebildkamera 3 und dem Gehirnareal 1 kann einen Dimensionierungsbereich zwischen 0,3m und 1 m umfassen.

Die Fig. 2 beschreibt schematisch den Weg der Bilddatenverarbeitung innerhalb der Bildverarbeitungseinheit 5. Die von der Wärmebildkamera 3 aufgenommenen Bilder werden fortlaufend in Form eines Datenkubus 10 in einem Bildspeicher (nicht eingezeichnet) abgespeichert. Nachfolgend wird eine Datenvorbehandlung 11 und nachfolgend die Klassifizierung der Bilddateninformationen 12 durchgeführt. Schließlich wird die Bildsequenz als berechnetes Bild dargestellt 13, wobei die funktionalen und/oder pathologischen Gehirnareale 1,18,19 hervorgehoben sind.

Die Fig. 3 zeigt die Anordnung des Aufnahmeverfahrens z.B. ohne Operationsmikroskop 7 während des neurochirurgischen Eingriffs am Gehirnareal 1. Die Wärmestrahlung 2 wird von der Wärmbildkamera 3 direkt aufgenommen und über eine Bildverarbeitungseinheit 5 einschließlich der Einheit 17 zur multivariaten Bildauswertung dem Monitor 6 zugeführt. Gleichzeitig können die mikroskopischen Bilder des Operationsmikroskop 7 mit eingespiegelt werden und/oder der zeitliche Verlauf 6' der Temperaturänderungen bestimmter Gehirnareale lässt sich zeitaufgelöst darstellen.

Das zugehörige Verfahren zur intraoperativen Bildgebung von Gehirnarealen 1, wobei mikroskopische Bilder und/oder präoperative Bilddaten auch registriert werden können,
weist erfindungsgemäß folgende Schritte auf
- die Oberfläche eines Gehirnareals 1 wird mittels einer Wärmebildkamera 3 bildhaft erfasst,
- die Wärmebilder 101, 102, 103, 104, 105, 106 werden einige Sekunden bis wenige Minuten kontinuierlich als Bildsequenz aufgenommen,
- die Bildsequenz wird einer Einheit 17 zur multivariaten Bildauswertung zugeführt und dort als Datenkubus 10 einer multivariaten Bildauswertung unterzogen und
- die temperaturunterschiedlichen Regionen 18, 19 des Gehirnareals 1 werden hervorgehoben,
- die Wärmebilder 101, 102, 103, 104, 105, 106 werden wahlweise in das jeweils zutreffende mikroskopische Bild eingespiegelt und/oder mit den präoperativen Bilddaten gemeinsam registriert.

Die Wärmebildkamera 3 kann so angebracht sein, dass sie unter Aufrechterhaltung der Sterilität in allen Raumrichtungen frei positioniert wird.

Es wird ausschließlich die gehirneigene Wärmestrahlung der Wärmebildkamera 3 zugeführt.

Es werden kontinuierlich Bilder von der abzubildenden Gehimoberfläche mit einer Abtastfrequenz zwischen 0.5HZ und 50Hz aufgenommen und gespeichert.

Der Abstand zwischen der Wärmebildkamera 3 und dem Gehirnareal 1 kann zwischen 0,3m und 1 m betragen.

Die Wärmebilder 101, 102, 103, 104, 105, 106 in Fig.3 werden sofort nach Abschluss der Aufnahmesequenz in einer Einheit zur multivariaten Bildauswertung ausgewertet und das berechnete Bild 13 wird ausgegeben.

Während der multivariaten Bildauswertung werden Störungen der Aufnahmen erkannt und die gestörten Wärmebilder aus dem Datenkubus 10 eliminiert.

Die Änderungen in der Basislinie werden automatisch korrigiert durch eine lineare oder nichtlineare Basislinienkorrektur kompensiert.

Die Wärmebilder 101, 102, 103, 104, 105, 106 können einer Hauptkomponentenanalyse, einem Verfahren der Clusteranalyse oder einem statistischen Klassifikationsverfahren zugeführt werden.

Die aktiven Gehirnareale 1 können in einem Falschfarbenbild deutlich hervortreten oder hervorgehoben werden.

Das Wärmebild 101, 102, 103, 104, 105, 106 kann einzeln oder fusioniert mit dem mikroskopischen Bild oder mit präoperativen Bilddaten dargestellt werden.

Die Bildsequenz der Wärmebilder 101, 102, 103, 104, 105, 106 können in zeitlicher Abfolge sichtbar gemacht werden.

Zusammenfassend können mittels des Verfahrens folgende Schritte vorgesehen werden:
- die aufgenommenen Wärmebilder 101, 102, 103, 104, 105, 106 werden einer Einheit 17 zur multivariaten Bildauswertung zugeführt und einem multivariaten Auswerteverfahren unterworfen, wobei ein Datenkubus 10 von Wärmebildem 101, 102, 103, 104, 105, 106 erstellt wird,
- Auswertung der Wärmebilder 101, 102, 103, 104, 105, 106 nach Abschluss der Wärmesequenz,
- Berechnung der auszugebenden Bilder 13, wobei Störungen in der Aufnahme erkannt werden und die gestörten Wärmebilder aus dem Datenkubus 10 entfernt werden,
- Korrektur der Änderungen in der Basislinie,
- Kompensation der Änderungen durch eine lineare oder eine nichtlineare Basislinienkorrektur, wobei das Wärmebild 101, 102, 103, 104, 105, 106 einzeln oder fusioniert mit dem mikroskopischen Bild oder mit präoperativen Bilddaten dargestellt wird.

Als Basislinie wird der Temperatur-Untergrund, d.h. ein konstanter Untergrund bei der kleinsten gemessenen Temperatur (z.B. 30°C) festgelegt, der sich durch eine langsame Veränderung der Temperatur infolge von äußeren Einflüssen (z.B. Erwärmung oder Abkühlung) oder durch die Wärmebildkamera 3 selbst infolge von Drift ändern kann. Innerhalb der relativ kurzen Messzeiten von wenigen Minuten sind die Änderungen in sehr guter Näherung linear und können durch eine Zwei-Punkt-Anpassung der Basislinie korrigiert werden.

Aufgezeichnete elektrophysiologische Messungen am abzubildenden Gehimareal 1 können in die Wärmebilder 101, 102, 103, 104, 105, 106 zeitgenau eingespiegelt werden.

Der zeitliche Ablauf von elektrophysiologischen Stimulationen kann zeitgenau mit den Wärmebildern 101, 102, 103, 104, 105, 106 dargestellt und beliebig oft wiederholbar gezeigt werden.

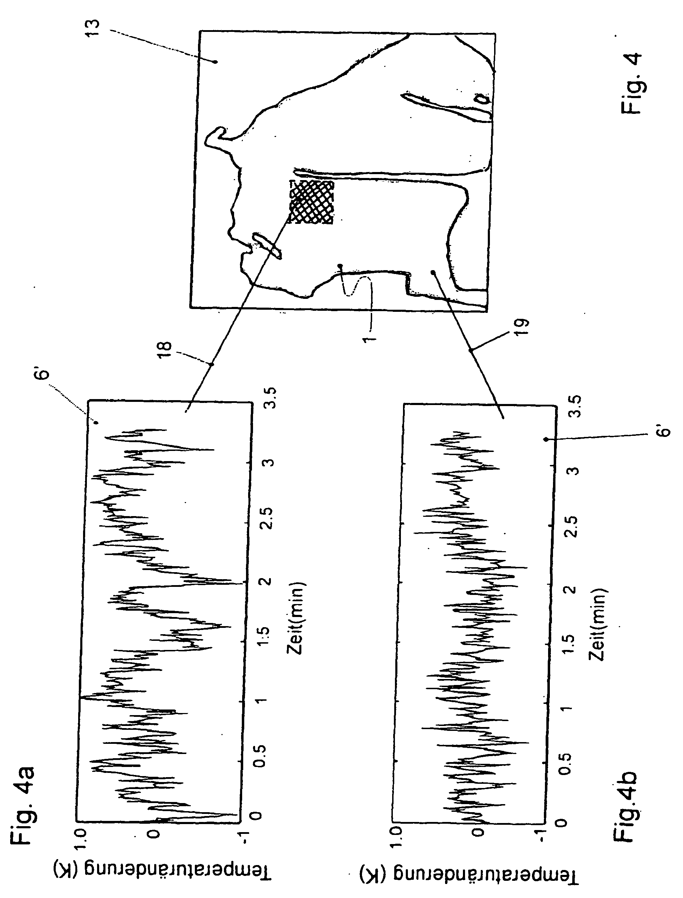

In der Fig. 4 ist ein Beispiel für die Lokalisierung von epileptischen Anfallsarealen dargestellt. Die in Fig. 4 dargestellten Temperatur-Zeit-Kurven als Aufnahmebilder von Oberflächenregionen 18, 19 des Gehirnareals 1 zeigen in Fig. 4a eine Oberflächenregion 18 mit erhöhter Temperatur und zugehörigem Temperaturverlauf und in Fig. 4b eine Oberflächenregion 19 mit normaler Temperatur und zugehörigem Temperaturverlauf. Die in dem berechneten Bild 13 schraffierten Regionen 18 weisen eine erhöhte Aktivität aus, was in dem zeitlichen Verlauf 6' der zugehörigen Temperaturänderung(K)-Zeit(min)-Kurve in Fig. 4a deutlich wird. Dagegen weisen die hellen Regionen 19 in dem Bild 13 eine deutlich geringere Aktivität aus, was sich ebenso in dem zeitlichen Verlauf 6' der zugehörigen Temperaturänderung(K)-Zeit(min)-Kurve in Fig. 4b erkennen lässt.

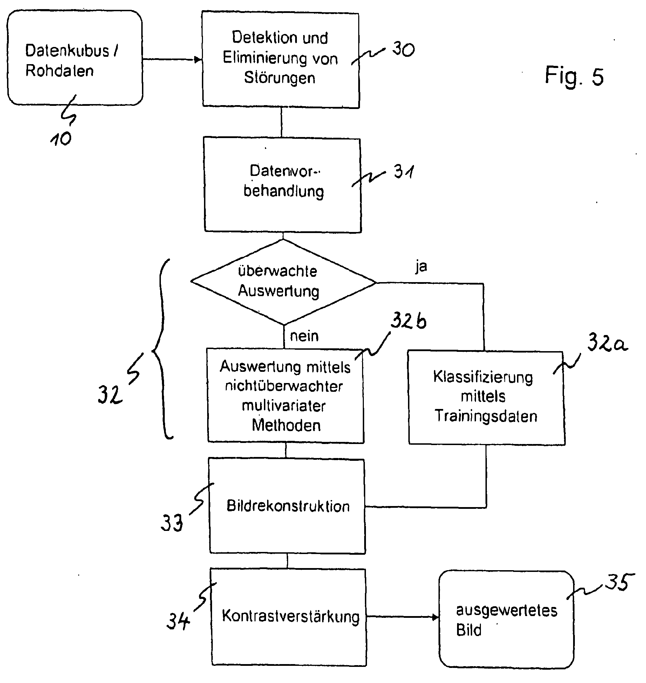

Die Fig. 5 zeigt den Ablauf der Bilddatenanalyse von Wärmebildern des Datenkubus 10. In einem ersten Schritt 30 werden alle Wärmebilder mit offensichtlichen Störungen herausgefiltert. Solche Störungen können durch Spülen der Gehirnoberfläche 1 oder durch kurzzeitige operative Eingriffe auftreten. In einem zweiten Schritt 31 werden pixelweise die zeitlichen Sequenzen einer Datenvorbehandlung unterzogen. Das umfasst zum Beispiel die Korrektur des Untergrundes bzw. der Basislinie oder die Normierung und Zentrierung auf einen bestimmten Parameter. Der dritte Schritt 32 umfasst die eigentliche multivariate Auswertung und Klassifizierung der Bilddaten. Dabei kann zwischen einem Verfahrenschritt mit überwachter Klassifizierung 32a und einem Verfahrensschritt mit nichtüberwachter Klassifizierung 32b gewählt werden. Es ist ebenso zweckmäßig, dass für bestimmte Anwendungen beide Verfahrensschritte 32a und 32b zum Einsatz kommen. Die ausgewerteten Daten werden dann in einem vierten Schritt 33 zu einem Bild 13 zusammengesetzt und schließlich in einem fünften Schritt 34 durch eine elektronische Kontrastverstärkung, zum Beispiel durch "digital staining", bearbeitet und im sechsten Schritt 35 wird das ausgewertete Bild 13 angezeigt.

Die Bildverarbeitungseinheit 5 kann einen Prozessor und zugehörige Speicher einschließlich Bildspeicher aufweisen, in denen die in der Bildanalyse vorgesehenen Schritte 30 bis 35 programmtechnisch unterstützt werden und die dazu eingesetzten Algorithmen als programmtechnische Mittel ausgeführt sind, die zur Durchführung des Berechnungsverfahrens der ausgegebenen/angezeigten und beobachtbaren Bilder 13 vorgesehen sind.

Eine Übersicht der derzeit möglichen Indikationen zur intraoperativen Thermographie führt für den speziellen Fall der Epilepsie auf:
Die therapieresistente Epilepsie bezeichnet ein Krankheitsbild, dass durch wiederholte, pharmakologisch nicht beherrschbare, äußerlich spontan auftretende Krampfanfälle gekennzeichnet ist. Häufig können mit den derzeitigen Möglichkeiten in diesen Fällen keine eindeutigen Läsionen zugeordnet werden. In solchen Fällen kann eine epilepsiechirurgische Operation zweckmäßig sein, in der zur weiteren Abklärung des Anfallsursprungs und der Anfallsausbreitung Gitterund Streifenelektroden auf die Gehimoberfläche gelegt werden, damit über mehrere Tage ein kortikales EEG (Elektroenzephalogramm) abgeleitet werden kann.

Ein epileptischer Krampfanfall ist die Folge paroxysmaler synchroner Entladungen von Neuronengruppen im Gehirn, die zu plötzlichen unwillkürlichen stereotypen Verhaltens- oder Befindensstörungen führen. Die Entladungen sind vermutlich von einer erhöhten metabolischen und vaskulären Antwort des betroffenen Gehirnareals 1 begleitet, die in einer veränderten Wärmeleistung, wie z.B. in der schraffierten Oberflächenregion 18 des Gehirnareals 1 in Fig. 4, resultieren können und mit einer thermographischen und örtlich hochauflösenden Wärmebildkamera 3 gemessen werden können.

Die Wärmebildkamera 3 kann dabei ungekühlt sein und zumindest eine Temperatur-Empfindlichkeit von 0,07 Kelvin aufweisen.

Es besteht aber bisher kein Verfahren, um den epileptogenen Focus und die durch Fortleitung übererregten Areale sichtbar zu machen, ohne dass gleichzeitig eine Anregung des Areals notwendig wäre, sei es durch sichtbare, UV- oder IR-Strahlung oder durch direkte Stimulation des Kortex.

Das erfindungsgemäße Thermographie-Verfahren stellt dagegen damit ein Verfahren dar, das - wie das EEG - allein auf den Unterschied von normalem Gewebe zu pathologischem Gewebe aufbaut und nicht auf zusätzliche Anregungen angewiesen ist, wie z.B. Fluorophor-basierten Darstellungen oder das im Stand der Technik genannte Optical Imaging.

Mit der Erfindung wird ein labelfreies, anregungsfreies, berührungsloses und rückwirkungsfreies Verfahren etabliert, das mit seiner technischen Ausführung übergangslos in den Ablauf einer neurochirurgischen Operation eingebettet werden kann.

### Bezugszeichenliste

1 Gehirnareal
2 Wärmestrahlungsbündel/Wärmestrahlung
3 Wärmebildkamera
4 Halterungsvorrichtung zur Befestigung und Führung der Wärmebildkamera
5 Bildverarbeitungseinheit der Wärmebilder
6 Videomonitor des Operationsmikroskops und der Wärmbilder
6' zeitliche Darstellung der Wärmestrahlung
7 Operationsmikroskop
8 EEG-Elektroden auf dem Gehirnareal
9 EEG-Gerät
10 Datenkubus von Wärmebildern
101 Wärmebild
102 Wärmebild
103 Wärmebild
104 Wärmebild
105 Wärmebild
106 Wärmebild
11 Einheit zur Entfernung von Störungen und Bildfehlern aus dem Datenkubus
12 Einheit zur multivariaten Analyse des Datenkubus
13 berechnetes Bild mit Informationen aus dem Datenkubus
14 sichtbares Licht
15 dichroitischer Teilerspiegel für sichtbares Licht und Wärmestrahlung
16 Spiegel für Wärmestrahlung
17 Einheit zur multivariaten Bildauswertung
18 Oberflächenregion mit höherer Temperatur
19 Oberrflächenregion mit normaler Temperatur
20 erfindungsgemäße Einrichtung
21 Linse
22 Linse
23 Spiegelsystem
30 erster Schritt: Herausfilterung von offensichtlichen Störungen aus den Wärmebildern des Datenkubus
31 zweiter Schritt: pixelweise Datenvorbehandlung von zeitlichen Sequenzen
32 dritter Schritt: Auswahl und Durchführung der multivariaten Auswertung und Klassifizierung der Bilddaten
32a Verfahrensschritt mit überwachter Klassifizierung
32b Verfahrensschritt mit nichtüberwachter Klassifizierung
33 vierter Schritt: Bildrekonstruktion
34 fünfter Schritt: Kontrastverstärkung
35 sechster Schritt: Ausgabe/Anzeige des ausgewerteten Bildes

## Patentansprüche

1. Verfahren zur intraoperativen Bildgebung von Gehirnarealen (1),
durch folgende Schritte gekennzeichnet:
- die Oberfläche eines Gehirnareals (1) wird mittels einer Wärmebildkamera (3) bildhaft erfasst, die einen Spektralbereich von 800nm bis zumindest 20000 nm erfasst und zugleich unempfindlich für sichtbares Licht ist und für die bei höheren Temperaturen des abzubildenden Gehirnareals (1) der Spektralbereich über 20000 nm hinausreicht,
- die Wärmebilder (101, 102, 103, 104, 105, 106) werden einige Sekunden bis wenige Minuten kontinuierlich als Bildsequenz aufgenommen,
- die Bildsequenz wird einer Einheit (17) zur multivariaten Bildauswertung zugeführt und dort als Datenkubus (10) einer multivariaten Auswertung unterzogen,
- temperaturunterschiedliche Bereiche (18, 19) der Oberfläche eines aktiven Gehirnareals (1) werden in einem Falschfarbenbild herausgehoben,
- die Wärmebilder (101, 102, 103, 104, 105, 106) werden auf ein mikroskopisches Bild überlagert und/oder mit präoperativen Bilddaten gemeinsam registriert,
wobei folgende Schritte durchgeführt werden
- Auswertung der Wärmebilder (101, 102, 103, 104, 105, 106) nach Abschluss der Wärmebildsequenz,
- Berechnung der auszugebenden Bilder (13), wobei Störungen in der Aufnahme erkannt werden und die gestörten Wärmebilder aus dem Datenkubus (10) entfernt werden,
- Korrektur der Änderungen in der Basislinie,
- Kompensation der Änderungen durch eine lineare oder nichtlineare Basislinienkorrektur, wobei das Wärmebild (101, 102, 103, 104, 105, 106) einzeln oder fusioniert mit dem mikroskopischen Bild oder mit präoperativen Bilddaten dargestellt wird, und
wobei der zeitliche Ablauf von elektrophysiologischen Stimulationen aus elektrophysiologischen Messungen am abzubildenden Gehirnareal (1) zeitgenau mit den Wärmebildern (101, 102, 103, 104, 105, 106) dargestellt und wiederholbar gezeigt werden.

2. Verfahren nach Anspruch 1,
**dadurch gekennzeichnet,**
**dass** die Wärmebildkamera (3) so angebracht wird, dass sie unter Aufrechterhaltung der Sterilität in allen Raumrichtungen frei positioniert wird.

3. Verfahren nach Anspruch 1,
**dadurch gekennzeichnet,**
**dass** ausschließlich die himeigene Wärmestrahlung der Wärmebildkamera (3) zugeführt wird.

4. Verfahren nach Anspruch 1,
**dadurch gekennzeichnet,**
**dass** mit einer Abtastfrequenz zwischen 0,5HZ und 50Hz kontinuierlich Bilder von der Oberfläche des Gehirnareals (1) aufgenommen und gespeichert werden.

5. Verfahren nach Anspruch 1 bis 4,
**dadurch gekennzeichnet,**
**dass** die Wärmebilder (101, 102, 103, 104, 105, 106) einer Hauptkomponentenanalyse, einem Verfahren der Clusteranalyse oder einem anderen statistischen Klassifikationsverfahren zugeführt werden.

6. Verfahren nach Anspruch 1,
**dadurch gekennzeichnet,**
**dass** die Bildsequenz der Wärmebilder (101, 102, 103, 104, 105, 106) in zeitlicher Abfolge sichtbar gemacht wird.

7. Verfahren nach mindestens einem der Ansprüche 1 bis 6,
**dadurch gekennzeichnet,**
**dass** zur Durchführung einer Wärmebildanalyse nach Erstellung des Datenkubus (10) von Wärmebildern (101, 102, 103, 104, 105, 106) folgende Schritte vorgesehen werden:
- Herausfilterung von offensichtlichen Störungen aus den Wärmebildern (101, 102, 103, 104, 105, 106) des Datenkubus (10) in einem ersten Schritt (30),
- pixelweise Datenvorbehandlung von zeitlichen Sequenzen in einem zweiten Schritt (31),
- Auswahl und Durchführung der multivariaten Auswertung und Klassifizierung der Bilddaten in einem dritten Schritt (32),
- Bildrekonstruktion in einem vierten Schritt (33),
- Kontrastverstärkung in einem fünften Schritt (34) und
- Ausgabe/Anzeige des ausgewerteten Bildes (13) in einem sechsten Schritt (35).

8. Verfahren nach Anspruch 7,
**dadurch gekennzeichnet,**
**dass** zur Wärmebildanalyse im dritten Schritt (32) der Auswertung und Klassifizierung eine überwachte Klassifizierung (32a) der Bilddaten mittels Trainingsdaten und/oder eine nichtüberwachte Klassifizierung (32b) durchgeführt werden.

9. Einrichtung (20) zur intraoperativen Bildgebung von Gehirnarealen (1), deren abgestrahlte Wärmestrahlung (2) mittels einer Detektion unter Einsatz des Verfahrens nach einem der vorhergehenden Ansprüche erfassbar ist,
wobei folgende Baugruppen zur Erfassung der Wärmestrahlung (2) vorhanden sind
- eine Wärmebildkamera (3) zur Aufnahme der vom Gehirnareal (1) abgestrahlten Wärmestrahlung in Form eines Wärmestrahlenbündels (2), wobei die Wärmebildkamera (3) einen Spektralbereich von 800 nm bis zumindest 20000 nm erfasst und zugleich unempfindlich für sichtbares Licht ist, wobei für höhere Temperaturen des abzubildenden Gehirnareals (1) der Spektralbereich über 20000 nm hinausreicht,
- eine Bildverarbeitungseinheit (5), die der Wärmebildkamera (3) nachgeordnet ist und in der die aufgenommenen Wärmebilder (101, 102, 103, 104, 105, 106) in einem Datenkubus (10) stapelartig gespeichert sind,
wobei in der Bildverarbeitungseinheit (5) eine Einheit (17) zur multivariaten Bildauswertung vorhanden ist, die
o eine Einheit (11) zur Entfernung von Störungen und Bildfehlern aus dem Datenkubus (10) und
o eine Einheit (12) zur multivariaten Analyse des Datenkubus (10) enthält,
wobei von der Bildverarbeitungseinheit (5) berechnete Bilder (13) und Informationen aus dem Datenkubus (10) der Wärmebilder (101, 102, 103, 104, 105, 106) zur Beobachtung erstellt werden, und
- Ausgabevorrichtungen (6, 6') für das Beobachten der Wärmebilder (101, 102, 103, 104, 105, 106),
wobei die Einrichtung (20)
- zumindest ein Operationsmikroskop (7), das ein Linsensystem (21, 22) zur Abbildung eines vorgesehenen Gehirnareals (1) mittels eines sichtbaren Lichtstrahlenbündels (14), das die vom Gehirnareal (1) abgestrahlte Wärmestrahlung als Wärmestrahlenbündel (2) enthält, aufweist,
- ein Spiegelsystem (23) mit zumindest einem dichroitischen Teilerspiegel (15) zur Transmission des sichtbaren Lichtstrahlenbündels (14) und zur Ablenkung des im Strahlengang des sichtbaren Lichtstrahlenbündels (14) befindlichen Wärmestrahlenbündels (2), wobei mittels des dichroitischen Teilerspiegels (15) das Wärmestrahlenbündel (2) aus dem sichtbaren Lichtstrahlenbündel (14) abgelenkt wird, und
- ein EEG-Gerät (9) für elektrophysiologische Messungen, wobei Elektroden des EEG-Gerätes (9) im Bereich des zu untersuchenden Gehirnareals (1) angebracht sind, mit denen der zugehörige EEG-Verlauf aufgezeichnet wird.

10. Einrichtung nach Anspruch 9,
**dadurch gekennzeichnet,**
**dass** das Spiegelsystem (23) mit dem die Wärmebilder (101, 102, 103, 104, 105, 106) ablenkenden dichroitischen Teilerspiegel (15) mindestens einen weiteren Spiegel (16) zur nachfolgenden Umlenkung des Wärmestrahlenbündels (2) zur Empfangsfläche der Wärmebildkamera (3) aufweist.

11. Einrichtung nach Anspruch 9 oder 10,
**dadurch gekennzeichnet,**
**dass** die Ausgabevorrichtungen (6, 6') zeitliche Darstellungen der Wärmebilder (101, 102, 103, 104, 105, 106) anzeigen.

12. Einrichtung nach Anspruch 9 oder 10,
**dadurch gekennzeichnet,**
**dass** der Datenkubus (10) von Wärmebildern eine Folge von zeitlich aufeinander folgend registrierten Wärmebildern (101, 102, 103, 104, 105, 106) aufweist.

13. Einrichtung nach Anspruch 9 oder 10,
**dadurch gekennzeichnet,**
**dass** in der Einheit (11) zur Entfernung von Störungen und Bildfehlern aus dem Datenkubus (10) folgende Funktionen realisiert sind:
- eine Clusteranalyse zur Trennung von gestörten und nichtgestörten Bildern,
- ein Filteralgorithmus, der Bilder mit einer physiologisch nicht möglichen schnellen Änderung in der Wärmestrahlung als Artefakt klassifiziert und eliminiert,
- ein Filter, der sämtliche Pixel eines Bildes unterhalb einer einzustellenden oder automatisch gewählten Temperaturgrenze aus dem Wärmebilddatensatz eliminiert,
- ein Rekonstruktionsalgorithmus, der vor allem flächenmäßig kleine Bildfehler durch mathematische Operationen ausgleicht.

14. Einrichtung nach Anspruch 9 oder 10,
**dadurch gekennzeichnet,**
**dass** in der Einheit (12) zur multivariaten Analyse des Datenkubus (10) folgende Funktionen realisiert sind:
- eine Hauptkomponentenanalyse,
- eine Koeffizientenanalyse,
- eine hierarchische oder nichthierachische Clusteranalyse,
- eine lineare Diskriminanzanalyse und ein Algorithmus zur Selektion relevanter Sequenzen,
- eine gestützte Klassifizierung unter Zuhilfenahme von Trainingsdaten, die in dem Algorithmus zur Selektion relevanter Sequenzen bereits gespeichert sind.

15. Einrichtung nach einem der vorhergehenden Ansprüche,
**dadurch gekennzeichnet,**
**dass** die Wärmebildkamera (3) ungekühlt ist und zumindest eine Temperatur-Empfindlichkeit von 0,07 Kelvin aufweist.

16. Einrichtung nach einem der vorhergehenden Ansprüche,
**dadurch gekennzeichnet,**
**dass** die Wärmebildkamera (3) separat an Haltevorrichtungen (4) oder an weiteren intraoperativen Instrumenten, an einer Navigationseinheit oder am Operationsmikroskop fixiert ist.

17. Einrichtung nach einem der vorhergehenden Ansprüche,
**dadurch gekennzeichnet,**
**dass** die Wärmebildkamera (3) in das Operationsmikroskop (7) integriert ist.

18. Einrichtung nach einem der vorhergehenden Ansprüche,
**dadurch gekennzeichnet,**
**dass** der Abstand zwischen der Wärmebildkamera (3) und dem Gehirnareal (1) einen Dimensionierungsbereich zwischen 0,3m und 1m umfasst.

## Claims

1. Method for intraoperative imaging of brain areas (1),
**characterized by** the following steps:
- an image of the surface of a brain area (1) is recorded with an infrared camera (3), which captures a spectral range of 800 nm to at least 20000 nm and is also nonsensitive to visible light, and for which the spectral range exceeds 20000 nm for higher temperatures of the imaged brain area (1),
- the thermal images (101, 102, 103, 104, 105, 106) are recorded continuously as image sequence for from several seconds up to a few minutes,
- the image sequence is fed into a unit (17) for multivariate image processing and subjected to a multivariate analysis as a data cube (10),
- areas with different temperatures (18, 19) of an active brain area (1) are highlighted in a false colour image,
- the thermal images (101, 102, 103, 104, 105, 106) are superimposed onto a microscopic image and/or registered together with pre-operative image data,
wherein the following steps are performed:
- analysis of the thermal images (101, 102, 103, 104, 105, 106) after completion of the thermal imaging sequence,
- calculation of the images to be output (13), wherein noise in the recording is detected and the noisy thermal images are removed from the data cube (10),
- correction of changes in the base line,
- compensation of changes with linear or non-linear base line correction, with the thermal image (101, 102, 103, 104, 105, 106) being presented individually or fused with the microscopic image or with pre-operative image data, and
wherein the temporal progress of electrophysiological stimulations from electrophysiological measurements at the brain area (1) to be imaged are presented exactly synchronised with the thermal images (101, 102, 103,104, 105, 106) and shown reliably.

2. Method according to Claim 1,
**characterized in that**
the infrared camera (3) is installed in such a way that it can be positioned freely in all spatial directions while maintaining sterility.

3. Method according to Claim 1,
**characterized in that**
the infrared camera (3) is only exposed to heat radiation emitted from the brain itself.

4. Method according to Claim 1,
**characterized in that**
a scanning frequency between 0.5 Hz and 50 Hz is used to continuously record and save images from the surface of the brain area (1).

5. Method according to Claims 1 to 4,
**characterized in that**
the thermal images (101, 102, 103, 104, 105, 106) are used in a principal component analysis, a procedure of cluster analysis or another statistical classification procedure.

6. Method according to Claim 1,
**characterized in that**
the image sequence of the thermal images (101, 102, 103, 104, 105, 106) is made visible in their temporal order.

7. Method according to at least one of the Claims 1 to 6,
**characterized in that**
the following steps are performed after creating the data cube (10) of the thermal images (101, 102, 103, 104, 105, 106) to carry out the analysis of thermal images:
- Filtering of obvious noise from the thermal images (101, 102, 103, 104, 105, 106) of the data cube (10) in a first step (30),
- Pixel-wise data pre-processing of temporal sequences in a second step (31),
- Selecting and performing the multivariate analysis and classification of the image data in a third step (32),
- Image reconstruction in a fourth step (33),
- Contrast enhancement in a fifth step (34), and
- Output/display of the analysed image (13) in a sixth step (35).

8. Method according to Claim 7,
**characterized in that**
a monitored classification (32a) of the image data using training data and/or
a non-monitored classification (32b) is performed for the analysis of thermal images in the third step (32) of the analysis.

9. Device (20) for intraoperative imaging of brain areas (1),
whose emitted heat radiation (2) can be recorded through detection using the method according to one of the preceding claims,
wherein the following assemblies are provided for recording of heat radiation (2)
- an infrared camera (3) to record heat radiation emitted by the brain area (1) in form of a heat radiation bundle (2), with the infrared camera (3) recording in a spectral range from 800 nm to at least 20000 nm, while also being nonsensitive to visible light, and with the spectral range exceeding 20000 nm for higher temperatures of the brain area (1) to be imaged,
- an image processing unit (5), which is connected downstream to the infrared camera (3) and in which the recorded thermal images (101, 102, 103, 104, 105, 106) are saved in a data cube (10) in the form of a stack, with a unit (17) for multivariate image processing being present in the image processing unit (5), which contains
o a unit (11) for the removal of noise and image errors from the data cube (10), and
o a unit (12) for the multivariate analysis of the data cube (10), wherein images (13) calculated by the image processing unit (5) and information of the thermal images (101, 102, 103, 104, 105, 106) from the data cube (10) are prepared for observation, and
- output devices (6, 6') for observing of the thermal images (101, 102, 103, 104, 105, 106),
wherein the device (20) comprises
- at least one surgical microscope (7) containing a lens system (21, 22) to image the target brain area (1) using a visible light radiation bundle (14) containing heat radiation emitted from the brain area (1) in the form of a heat radiation bundle (2),
- a mirror system (23) with at least one dichroic beam splitting mirror (15) for transmission of the visible light radiation bundle (14) and for deflection of the heat radiation bundle (2) located inside the optical path of the visible light radiation bundle (14) with the heat radiation bundle (2) being deflected from the visible light radiation bundle (14) using the dichroic beam splitting mirror (15), and
- an EEG device (9) for electrophysiological measurements wherein electrodes of the EEG device (9) are applied to the brain area (1) to be examined, which are used to record the associated EEG curve.

10. Device according to Claim 9,
**characterized in that**
the mirror system (23) with the dichroic beam splitting mirror (15) deflecting
the thermal images (101, 102, 103, 104, 105, 106) comprises at least one further mirror (16) for subsequent deflection of the heat radiation bundle (2) to the receiving surface of the infrared camera (3).

11. Device according to Claim 9 or 10,
**characterized in that**
the output devices (6, 6') display temporal representations of the thermal images (101, 102, 103, 104, 105, 106).

12. Device according to Claim 9 or 10,
**characterized in that**
the data cube (10) of the thermal images (101, 102, 103, 104, 105, 106) exhibits a sequence of thermal images registered subsequently in time.

13. Device according to Claim 9 or 10,
**characterized in that**
the following functions are realised in the unit (11) for removal of noise and image error from the data cube (10):
- a cluster analysis for separation of images with and without noise,
- a filter algorithm, which classifies and eliminates images with a physiologically impossibly fast change of the heat radiation as artefacts,
- a filter, which eliminates all pixels of an image below the temperature threshold to be set or selected automatically from the set thermal images,
- a reconstruction algorithm, which mainly balances small area image errors with mathematical operations.

14. Device according to Claim 9 or 10,
**characterized in that**
the following functions are realised in the unit (12) for multivariate analysis of the data cube (10):
- a principle components analysis,
- a coefficient analysis,
- a hierarchical or non-hierarchical cluster analysis,
- a linear discriminant analysis and an algorithm for selection of relevant sequences,
- a supported classification using training data, which are already saved in the algorithm for selection of relevant sequences.

15. Device according to one of the preceding claims,
**characterized in that**
the infrared camera (3) is uncooled and exhibits a temperature sensitivity of at least 0.07 Kelvin.

16. Device according to one of the preceding claims,
**characterized in that**
the infrared camera (3) is fixed separately to a holding device (4) or another operative instrument, to a navigation unit or to the surgical microscope.

17. Device according to one of the preceding claims,
**characterized in that**
the infrared camera (3) being integrated in the surgical microscope (7).

18. Device according to one of the preceding claims,
**characterized in that**
the distance between the infrared camera (3) and the brain area (1) has a dimensional range between 0.3 m und 1 m.

## Revendications

1. Procédé d'imagerie intraopératoire de zones du cerveau (1) **caractérisé par** les étapes suivantes:
- on saisit la surface d'une zone du cerveau (1) en image au moyen d'une caméra thermique (3), qui saisit une plage spectrale entre 800nm et au moins 20000 nm et qui en plus est insensible à la lumière visible, la plage spectrale dépassant 20000 nm pour des températures plus élevées de la zone du cerveau (1) à représenter,
- on enregistre en continu les images thermiques (101, 102, 103, 104, 105, 106), pendant une durée de quelques secondes à quelques minutes sous forme de séquence d'images,
- on fournit la séquence d'images à une unité (17) d'évaluation multivariée d'images et on la soumet en tant que cube de données (10) à une évaluation multivariée,
- on met en avant des zones de températures différentes (18, 19) de la surface d'une zone active du cerveau (1) dans une image en fausses couleurs,
- on superpose les images thermiques (101, 102, 103, 104, 105, 106) sur une image microscopique et/ou enregistrées ensemble avec des données préopératoires,
procédé selon lequel
- on évalue les images thermiques (101, 102, 103, 104, 105, 106) après la fin de la séquence d'images thermiques,
- on calcule des images à éditer (13), les défauts sur la prise de vue étant reconnus et les images thermiques défectueuses étant retirées du cube de données (10),
- on corrige les modifications dans la ligne de base,
- on compense les modifications par une correction de ligne de base linéaire ou non-linéaire, l'image thermique (101, 102, 103, 104, 105, 106) étant représentée individuellement ou fusionnée avec l'image microscopique ou avec les données d'images préopératoires, et
on présente le déroulement chronologique des stimulations électrophysiologiques des mesures électrophysiologiques sur la zone du cerveau (1) à représenter et on l'affiche de façon à pouvoir le répéter en même temps que les images thermiques (101, 102, 103, 104, 105, 106).

2. Procédé suivant la revendication 1
**caractérisé en ce que**
on installe et on positionne librement la caméra thermique (3) dans toutes les directions de l'espace sans rompre la stérilité.

3. Procédé suivant la revendication 1
**caractérisé en ce que**
on applique seulement le rayonnement thermique propre au cerveau à la caméra thermique (3).

4. Procédé suivant la revendication 1
**caractérisé en ce que**
on prend des images de la surface de la zone du cerveau (1) à représenter et on les enregistre en continu avec une fréquence de balayage entre 0,5HZ et 50HZ.

5. Procédé suivant les revendications 1 à 4
**caractérisé en ce que**
on soumet les images thermiques (101, 102, 103, 104, 105, 106) à une analyse de composant principale, à un procédé d'analyse de partitionnement des données ou à un autre procédé de classification statique.

6. Procédé suivant la revendication 1
**caractérisé en ce que**
on rend visible la séquence des images thermiques (101, 102, 103, 104, 105, 106) selon une suite chronologique.

7. Procédé suivant au moins l'une des revendications 1 à 6
**caractérisé en ce que**
pour faire l'analyse d'images thermiques après avoir établi le cube de données (10) des images thermiques (101, 102, 103, 104, 105, 106), on effectue les étapes suivantes :
- dans une première étape (30) on élimine par filtrage des défauts apparents des images thermiques (101, 102, 103, 104, 105, 106) du cube de données (10),
- dans une deuxième étape (31) on fait un pré-traitement des données pixel par pixel de séquences temporelles,
- dans une troisième étape (32) on sélectionne et on exécute l'évaluation multivariée et la classification des données d'image,
- dans une quatrième étape (33) on reconstruit l'image,
- dans une cinquième étape (34) on amplifie les contrastes et
- dans une cinquième étape (34) on édite/affiche l'image évaluée (13).

8. Procédé suivant la revendication 7
**caractérisé en ce que**
pour l'analyse de l'image thermique à la troisième étape (32) de l'évaluation et de la classification, on effectue une classification surveillée (32a) des données d'image avec des données d'apprentissage et/ou une classification non-surveillée (32b).

9. installation (20) d'imagerie intraopératoire de zones du cerveau (1), dont le rayonnement thermique (2) émis est saisi par une détection selon le procédé de l'une des revendications précédentes, en utilisant les éléments suivants pour saisie du rayonnement thermique (2)
- une caméra thermique (3) pour la prise de vue du rayonnement thermique émis par la zone du cerveau (1) sous la forme d'un faisceau de rayonnement thermique (2), la caméra thermique (3) saisissant une zone spectrale comprise entre 800nm et au moins 20000 nm, et étant en plus insensible à la lumière visible, la zone spectrale dépassant 20000 nm pour des températures plus élevées de la zone du cerveau (1) à représenter,
- une unité de traitement de l'image (5) en aval de la caméra thermique (3) et qui enregistre en pile, les images thermiques (101, 102, 103, 104, 105, 106) dans un cube de données (10), l'unité de traitement de l'image (5) ayant une unité (17) pour évaluation multivarée de l'image, se composant
o d'une unité (11) pour éliminer les défauts et les erreurs d'image du cube de données (10) et
o d'une unité (12) pour effectuer une analyse multivarée du cube de données (10),
les images (13) calculées par l'unité de traitement de l'image (5) et les informations à partir du cube de données (10) des images thermiques (101, 102, 103, 104, 105, 106) étant réalisées à des fins d'observation et
- des dispositifs d'édition (6, 6') pour observer les images thermiques (101, 102, 103, 104, 105, 106) l'installation (20) ayant
- au moins un microscope d'opération (7) avec un système de lentilles (21, 22) pour représenter la zone du cerveau (1) prévue, à l'aide du faisceau (14) de lumière visible qui contient le rayonnement thermique émis par la zone du cerveau (1) à représenter, comme faisceau de rayonnement thermique (2),
- un système de miroirs (23) avec au moins un miroir semi-réfléchissant dichronique (15) pour transmettre le faisceau lumineux (14) visible et dévier le faisceau de rayonnement thermique (2) du trajet du faisceau lumineux (14) visible, le faisceau de rayonnement thermique (2) étant dévié du faisceau lumineux (14) par le miroir semi-réfléchissant dichroïque (15), et
- un appareil EEG (9) pour des mesures électrophysiologiques, dont les électrodes, pour enregistrer la courbe EEG, étant placées dans la zone du cerveau (1) examinée.

10. Installation suivant la revendication 9,
**caractérisée en ce que**
le système de miroirs (23) avec le miroir séparateur dichroïque (15) déviant les images thermiques (101, 102, 103, 104, 105, 106) a au moins un autre miroir (16) pour ensuite dévier le faisceau de rayonnement thermique (2) vers la surface de réception de la caméra thermique (3).

11. Installation suivant la revendication 9 ou 10,
**caractérisé en ce que**
les dispositifs d'édition (6, 6') affichent des représentations chronologiques des images thermiques (101, 102, 103, 104, 105, 106).

12. Installation suivant la revendication 9 ou 10,
**caractérisée en ce que**
le cube de données (10) d'images thermiques a une séquence d'images thermiques (101, 102, 103, 104, 105, 106) enregistrées chronologiquement l'une à la suite de l'autre.

13. Installation suivant la revendication 9 ou 10,
**caractérisée en ce que**
pour éliminer les défauts et les erreurs d'image du cube de données (10), l'unité (11) effectue les fonctions suivantes :
- une analyse de partitionnement des données pour séparer les images défectueuses et celles non défectueuses,
- un algorithme de filtrage classant et éliminant comme artefacts, des images à modification rapide, physiologiquement impossible, du rayonnement thermique,
- un filtre éliminant tous les pixels d'une image sous le seuil de température à paramétrer ou sélectionnés automatiquement à partir du groupe de données d'images thermiques,
- un algorithme de reconstruction qui compense, d'abord sur toute la surface, les petits défauts des images par des opérations mathématiques.

14. Installation suivant la revendication 9 ou 10,
**caractérisée en ce que**
l'unité (12) d'analyse multivariée du cube de données (10), exécute les fonctions suivantes :
- une analyse des composants principaux,
- une analyse des coefficients,
- une analyse de partitionnement des données hiérarchique ou non-hiérarchique,
- une analyse discriminante linéaire et un algorithme de sélection de séquences pertinentes,
- une classification assistée en recourant aux données d'apprentissage déjà stockées dans l'algorithme de sélection de séquences pertinentes.

15. Installation suivant l'une des revendications précédentes,
**caractérisée en ce que**
la caméra thermique (3) n'est pas refroidie et présente une sensibilité à la température d'au moins 0,07 kelvin.

16. Installation suivant l'une des revendications précédentes,
**caractérisée en ce que**
la caméra thermique (3) est fixée séparément aux dispositifs de maintien (4) ou à d'autres instruments intraopératoires, à une unité de navigation ou au microscope d'opération.

17. Installation suivant l'une des revendications précédentes,
**caractérisée en ce que**
la caméra thermique (3) est intégrée dans le microscope d'opération (7).

18. Installation suivant l'une des revendications précédentes,
**caractérisée en ce que**
la distance entre la caméra thermique (3) et la zone du cerveau (1) est comprise entre 0,3m et 1 m.
